# EUROPEAN PATENT SPECIFICATION

(11) **EP 3 600 101 B1**
(45) Date of publication and mention of the grant of the patent: **17.06.2020**
(21) Application number: 18718575.6
(22) Date of filing: 16.03.2018
(51) Int. Cl.: A61B 18/12, A61B 5/055, A61N 7/02, G01R 33/48

(54) **LOCATING ABLATED TISSUES USING ELECTRIC PROPERTIES TOMOGRAPHY**
ORTUNG ABLATIERTER GEWEBE MITTELS ELEKTRISCHER MERKMALSTOMOGRAPHIE
LOCALISATION DE TISSUS ENLEVÉS À L'AIDE DE LA TOMOGRAPHIE DE PROPRIÉTÉS ÉLECTRIQUES

(30) Priority: 20.03.2017 EP 17161821
(43) Date of publication of application: 05.02.2020
(73) Proprietor: Koninklijke Philips N.V., 5656 AG Eindhoven (NL)
(72) Inventor: KATSCHER, Ulrich, 5656 AE Eindhoven (NL); KEUPP, Jochen, 5656 AE Eindhoven (NL); GRUELL, Holger, 5656 AE Eindhoven (NL); HEIJMAN, Edwin, 5656 AE Eindhoven (NL); YEO, Sin Yuin, 5656 AE Eindhoven (NL)
(74) Representative: Philips Intellectual Property & Standards
(86) International application number: PCT/EP2018/056617
(87) International publication number: WO 2018/172195

(56) References cited:
- EP-A1- 2 636 368
- WO-A1-2016/116545
- JP-A- 2000 300 535
- US-A1- 2015 051 475
- OH IN KWON ET AL: "Fast conductivity imaging in magnetic resonance electrical impedance tomography (MREIT) for RF ablation monitoring", INTERNATIONAL JOURNAL OF HYPERTHERMIA, vol. 30, no. 7, 20 October 2014 (2014-10-20), pages 447-455, XP055406098, GB ISSN: 0265-6736, DOI: 10.3109/02656736.2014.966337 cited in the application

## Description

### FIELD OF THE INVENTION

The invention relates to magnetic resonance imaging, in particular to electric properties tomography.

### BACKGROUND OF THE INVENTION

Thermal tissue ablation may be used to destroy diseased or cancerous tissue by heating. One example of tissue ablation is high intensity focused ultrasound (HIFU). In HIFU an array of ultrasonic transducer elements are used to form an ultrasonic transducer. Supplying alternating current electrical power to the transducer elements causes them to generate ultrasonic waves. The ultrasonic waves from each of the transducer elements either add constructively or destructively. By controlling the phase of alternating current electrical power supplied to each of the transducer elements the focal point or volume into which the ultrasound power is focused may be controlled.

The journal article Kwon, Oh In, et al. "Fast conductivity imaging in magnetic resonance electrical impedance tomography (MREIT) for RF ablation monitoring." International Journal of Hyperthermia 30.7 (2014): 447-455 (herein referred to as "Kwon et. al.") discloses the detection of structural changes in tissue during RF ablation using a fast MREIT conductivity imaging method. Document US2015/0051475 discloses a magnetic resonance imaging system to determine a temperature map using the B1 field magnetic resonance data.

### SUMMARY OF THE INVENTION

The invention provides for a medical system, a computer program product, and a method in the independent claims. Embodiments are given in the dependent claims.

Various methods exist for thermally ablating tissue. A difficulty in using these method effectively is that it can be difficult to determine what tissue has been successfully ablated. This information may be useful in assessing a treatment after it has occurred or even as part of the control of such a therapy. Embodiments may provide for an improved means of detecting tissue that has been thermally ablated. Embodiments may do this by using a technique referred to as Magnetic Resonance Electric Properties Tomography ("MR EPT" or "EPT"). In EPT, the conductivity and/or permittivity can both be determined as a function of position within the subject by solving the homogeneous Helmholtz equation.

Embodiments are able to detect thermally ablated tissue by detecting a change in the conductivity and/or permittivity using EPT. The EPT technique is non invasive and measures the conductivity and/or permittivity at a high frequency. The MREIT technique described in Kwon et. al. relies on electrode inserted within a subject in inject DC currents into the subject to measure a DC current. The conductivity change detected by the MREIT technique is equivalent to a DC or low frequency measurement of the conductivity. The conductivity change measured by the MREIT technique is therefore mostly dependent upon the structural change in the cells, i.e. the destruction of cell membranes and other structures. The EPT technique makes a radio frequency (RF) measurement of an RF electrical property and this measurement is more sensitive to chemical changes induced by thermal ablation. The EPT technique may indicate regions that will eventually die due to necrosis that have not experienced massive changes in cellular structure.

In one aspect the invention provides for a medical system comprising a memory for storing machine-executable instructions. The medical system further comprises a processor for controlling the medical system. A memory as used herein may encompass one or more memories. A processor as used herein may also encompass one or more processors within the same machine or distributed within a group or different machines.

Execution of the machine-executable instructions cause the processor to receive first electric properties tomography data descriptive of a first spatially dependent mapping of a RF electrical property within a region of interest of the subject. A radio frequency (RF) electrical property, as used herein is understood to encompass an electrical property of substance or region at a frequency of 1 MHz or greater (and less than 300 GHz). Alternatively, an RF electrical property can also be understood to be an electrical property of a substance or a region at a frequency of 10 MHz or greater (and less than 500 MHz). A region of interest as used herein encompasses a volume of the subject. The RF electrical property is a real valued permittivity or a real valued conductivity. The first electric properties tomography data may be the first spatially dependent mapping itself or it may be data which can be used to calculate the first spatially dependent mapping of the RF electrical property. For example the electric properties tomography data may be data acquired from a magnetic resonance imaging system that uses an EPT or electric properties tomography pulse sequence commands for acquiring magnetic resonance data. The electric properties tomography data as used herein may therefore in some examples be magnetic resonance data. Execution of the machine-executable instructions further cause the processor to receive second electric properties tomography data descriptive of a second spatially dependent mapping of the spatially dependent RF electrical property within the region of interest of the subject. Execution of the machine-executable instructions further cause the processor to calculate a change in the spatially dependent RF electrical property derived from a difference between the first electric properties tomography data and the second electric properties tomography data. In different examples this may be calculated differently.

In some examples, the change in the spatially dependent RF electrical property is calculated from the first spatially dependent mapping and the second spatially dependent mapping. In this case the first spatially dependent mapping is calculated from the first electrical properties tomography data and the second spatially dependent mapping is calculated from the second electrical properties tomography data. In other cases the change in the spatially dependent RF electrical property can be derived or calculated directly from the first electric properties tomography data and the second electric properties tomography data.

Execution of the machine-executable instructions further cause the processor to calculate a spatially dependent ablation map by indicating regions within the region of interest where the change in the spatially dependent RF electrical property is above a predetermined threshold. This embodiment may be beneficial because when tissue is ablated thermally there may be large changes in either the real valued conductivity and/or the real valued permittivity. This embodiment may facilitate an easy way of determining ablated tissue.

In another embodiment, the spatially dependent ablation map is superimposed or correlated to a magnetic resonance image. This for example may enable the superimposing of the spatially dependent ablation map on an image which assists a medical technician or a physician in interpreting the spatially dependent ablation map.

When performing electrical properties tomography using a magnetic resonance imaging system there may be three different ways to determine the complex permittivity. The first may be to estimate conductivity by measuring and then post-processing only the B1 phase. Another way is to estimate the non-complex permittivity by measuring and post-processing only data from a B1 magnitude image. A third way to exactly determine conductivity and the non-complex permittivity by measuring and post-processing B1 magnitude and B1 phase data.

In another embodiment, the predetermined threshold is a 5% change.

In another embodiment, the predetermined threshold is a 10% change.

In another embodiment; the predetermined threshold is a 20% change.

In another embodiment; execution of the machine-executable instructions further cause the processor to receive a first spatially dependent temperature map of the region of interest for the first electric properties tomography data. Execution of the machine-executable instructions further cause the processor to receive a second spatially dependent temperature map of the region of interest for the second electric properties tomography data. The change in the spatially dependent RF electrical property is temperature corrected using a change between the first spatially dependent temperature map and the second spatially dependent temperature map. This embodiment may be beneficial because the real valued permittivity or the real valued conductivity change as a function of temperature. In practice a linear model with a 2% correction per degree Celsius works well in calculating changes in conductivity due to temperature. Also in practice a linear model correcting the permittivity with a -0.5% per degree change works well also.

In the above embodiment; it is not necessarily required to calculate absolute temperatures. As the temperature change between the two time periods when the first electrical properties tomography data was acquired and when the second electrical properties tomography data was acquired is all that is necessary. The first spatially dependent temperature map and the second spatially dependent temperature map need not be calibrated to an exact scale so long as they have a calibration which is correct relative to each other.

In another embodiment; the medical system further comprises a magnetic resonance imaging system. The memory further stores EPT or electrical properties tomography pulse sequence commands configured for controlling the magnetic resonance imaging system to acquire the first electrical properties tomography data and the second electrical properties tomography data according to an electrical properties tomography magnetic resonance imaging protocol. The first electrical properties tomography data is received by controlling the magnetic resonance imaging system with the EPT pulse sequence commands. The second electrical properties tomography data is received by controlling the magnetic resonance imaging system with the EPT pulse sequence commands.

This embodiment may be beneficial because it may provide for an efficient means of looking at ablated tissue using only a magnetic resonance imaging system. When only using a magnetic resonance imaging system by itself it may not be necessary to do temperature correction when calculating the spatially dependent ablation map. For example a subject could have a magnetic resonance image that is acquired before a thermal ablation procedure is performed. The subject may then be removed from the magnetic resonance imaging system and then have an ablation of tissue performed using any of a variety of different techniques. The subject could then be placed back into the magnetic resonance imaging system and the data for the second electric properties tomography data could be acquired. There may also be a substantial time change on the order of hours or maybe even a day or two between when the data may be acquired. In some instances a long delay may be beneficial because the temperature of the subject within the region of interest may return to the subject's normal or core body temperature.

In another embodiment, the magnetic resonance imaging system has an imaging zone. The medical system further comprises a tissue heating system for heating a target zone within the imaging zone. In other words, the tissue heating system is configured such that it is able to heat a target zone within the imaging zone. The tissue heating system is configured for heating within the region of interest between the acquisition of the first electrical properties tomography data and the second electrical properties tomography data. This embodiment may be advantageous because it may provide for a means of directly measuring the amount or volume of tissue ablated by the tissue heating system.

In another embodiment, the tissue heating system is a high-intensity focused ultrasound heating system.

In another embodiment, the tissue heating system is a radio-frequency heating system. There may for example be a radio-frequency generator and one or more antennas which may be used for locally heating the target zone.

In another embodiment, the tissue heating system is a microwave ablation system. For example there may be a microwave applicator or probe which can be used to locally heat the target zone within the subject.

In another embodiment, the tissue heating system is a hypothermia therapy system. For example air or fluids or a heated surface may be used to heat a portion of the subject.

In another embodiment, the tissue heating system is a laser ablation system.

In another embodiment, the tissue heating system is an infrared ablation system.

In another embodiment, the tissue heating system is a high-intensity focused ultrasound system with a controllable focus for causing ultrasonic energy within the target zone. The memory further comprises sonication commands for controlling targeting of the controllable focus. The sonication commands are configured for controlling the high-intensity focused ultrasound system to sonicate the target zone in discreet sonication periods separated by cooling periods. Execution of the machine-executable instructions further causes the processor to repeatedly acquire the first electrical properties tomography data and the second electric properties tomography data during at least a portion of the cooling periods. This embodiment may be beneficial because it may provide for a means of measuring ablated tissue during the cooling periods.

In some examples, during the cooling periods the magnetic resonance imaging system may also be configured for acquiring the first thermal magnetic resonance data and the second thermal magnetic resonance data. This may be beneficial as it may provide for a more accurate ablation map.

In another embodiment, execution of the machine-executable instructions further cause the processor to modify the sonication commands using the spatially dependent ablation map after acquisition of the first electric properties tomography data and the second electric properties tomography data. This may be beneficial because the determination of the spatially dependent ablation map may be used to provide direct feedback in controlling the high-intensity focused ultrasound system. The ablation map may be used to form a closed control loop.

In another embodiment, the memory further stores temperature sensitive pulse sequence commands configured for controlling the magnetic resonance imaging system to acquire first thermal magnetic resonance data and second thermal magnetic resonance data according to a magnetic resonance imaging thermometry protocol.

Execution of the machine-executable instructions further cause the processor to control the magnetic resonance imaging system to acquire the first thermal magnetic resonance data using the temperature sensitive pulse sequence commands. The first thermal magnetic resonance data is acquired within a predetermined period of when the first electrical properties tomography data is acquired. The first spatially dependent temperature map is received by reconstructing the first spatially dependent temperature map from the first thermal magnetic resonance data. Execution of the machine-executable instructions further cause the processor to control the magnetic resonance imaging system to acquire the second thermal magnetic resonance data using the temperature sensitive pulse sequence commands. The second thermal magnetic resonance data is acquired within the predetermined period of when the second electrical properties tomography data is acquired. The second spatially dependent temperature map is received by reconstructing the second spatially dependent temperature map from the second thermal magnetic resonance data. This embodiment may be beneficial because it may provide for a means of correcting the ablation map.

In some examples, the predetermined period of when the first electrical properties tomography data is acquired is a cooling period immediately after when the first electrical properties tomography data is acquired. In this example the predetermined period of when the second electrical properties tomography data is acquired may be a cooling period immediately after the second electrical properties tomography data is acquired. In some instances the first and second thermal magnetic resonance data may be acquired in an interleaved fashion with the first and second electric properties tomography data respectively.

When performing magnetic resonance thermometry there may be for example baseline measurements which are performed initially. For the above embodiment having the absolute temperature calibration is however not necessary. As the change in the conductivity and the permittivity can be effectively modeled using a linear model the change in the temperature is the relevant value for correcting the ablation map. A calibration for the temperature measurements to determine absolute temperature values is therefore not necessary.

In another aspect, the invention provides for a method of operating the medical system. The method comprises receiving first electric properties tomography data descriptive of the first spatially dependent mapping of an electric property within the region of interest of a subject. The electric property is a real valued permittivity or a real valued conductivity. The method further comprises receiving second electric properties tomography data descriptive of a second spatially dependent mapping of the spatially dependent electric property within the region of interest. The method further comprises calculating a change in the spatially dependent RF electrical property derived from the difference between the first electrical properties tomography data and the second electrical properties tomography data. The method further comprises calculating a spatially dependent ablation map by indicating regions within the region of interest or identifying regions where the change in the spatially dependent RF electrical property is above a predetermined threshold.

In another aspect, the invention provides for a computer program product comprising machine-executable instructions for execution by a processor controlling the medical instrument. Execution of the machine-executable instructions causes the processor to receive first electric properties tomography data descriptive of a first spatially dependent mapping of an electric property within a region of interest of the subject. The electric property is a real valued permittivity or a real valued conductivity. Execution of the machine-executable instructions further cause the processor to receive second electric properties tomography data descriptive of a second spatially dependent mapping of the spatially dependent RF electrical property within the region of interest of the subject.

Execution of the machine-executable instructions further cause the processor to calculate a change in the spatially dependent RF electrical property derived from a difference between the first electrical properties tomography data and the second electrical properties tomography data. Execution of the machine-executable instructions further cause the processor to calculate a spatially dependent ablation map by indicating regions within the region of interest where the change in the spatially dependent RF electrical property is above a predetermined threshold.

In another embodiment, the medical system further comprises a magnetic resonance imaging system. The first electrical properties tomography data is received by controlling the magnetic resonance imaging system with pulse sequence commands. The EPT pulse sequence commands are configured for controlling the magnetic resonance imaging system to acquire the first electric properties tomography data and the second electrical properties tomography data according to an electrical properties tomography magnetic resonance imaging protocol. The second electrical properties tomography is received by controlling the magnetic resonance imaging system with EPT pulse sequence commands.

In another embodiment, the medical system further comprises a high-intensity focused ultrasound system with a controllable focus for depositing ultrasonic energy within the target zone. The target zone is within the imaging zone. The high-intensity focused ultrasound is configured for heating within the region of interest between the acquisition of the first electrical properties tomography data and the second electrical properties tomography data. The memory further comprises sonication commands for controlling targeting of the controllable focus. The sonication commands may be part of the machine-executable instructions. The sonication commands are configured for controlling the high-intensity focused ultrasound system to sonicate the target zone in discreet sonication periods separated by cooling periods. Execution of the machine-executable instructions further causes the processor to repeatedly acquire the first electrical properties tomography data and the second electric properties tomography data during at least a portion of the cooling periods.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage may be any volatile or non-volatile computer-readable storage medium.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the C programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, bluetooth connection, wireless local area network connection, TCP/IP connection, ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) display, Electroluminescent display (ELD), Plasma display panel (PDP), Liquid crystal display (LCD), Organic light-emitting diode display (OLED), a projector, and Head-mounted display.

Magnetic Resonance (MR) data is defined herein as being the recorded measurements of radio frequency signals emitted by atomic spins using the antenna of a magnetic resonance apparatus during a magnetic resonance imaging scan. Magnetic resonance data is an example of medical imaging data. A Magnetic Resonance (MR) image is defined herein as being the reconstructed two or three dimensional visualization of anatomic data contained within the magnetic resonance imaging data.
Magnetic resonance data may comprise the measurements of radio frequency signals emitted by atomic spins by the antenna of a Magnetic resonance apparatus during a magnetic resonance imaging scan which contains information which may be used for magnetic resonance thermometry. Magnetic resonance thermometry functions by measuring changes in temperature sensitive parameters. Examples of parameters that may be measured during magnetic resonance thermometry are: the proton resonance frequency shift, the diffusion coefficient, or changes in the T1 and/or T2 relaxation time may be used to measure the temperature using magnetic resonance. The proton resonance frequency shift is temperature dependent, because the magnetic field that individual protons, hydrogen atoms, experience depends upon the surrounding molecular structure. An increase in temperature decreases molecular screening due to the temperature affecting the hydrogen bonds. This leads to a temperature dependence of the proton resonance frequency.

The proton density depends linearly on the equilibrium magnetization. It is therefore possible to determine temperature changes using proton density weighted images.

The relaxation times T1, T2, and T2-star (sometimes written as T2*) are also temperature dependent. The reconstruction of T1, T2, and T2-star weighted images can therefore be used to construct thermal or temperature maps.

The temperature also affects the Brownian motion of molecules in an aqueous solution. Therefore pulse sequences which are able to measure diffusion coefficients such as a pulsed diffusion gradient spin echo may be used to measure temperature.

One of the most useful methods of measuring temperature using magnetic resonance is by measuring the proton resonance frequency (PRF) shift of water protons. The resonance frequency of the protons is temperature dependent. As the temperature changes in a voxel the frequency shift will cause the measured phase of the water protons to change. The temperature change between two phase images can therefore be determined. This method of determining temperature has the advantage that it is relatively fast in comparison to the other methods. The PRF method is discussed in greater detail than other methods herein. However, the methods and techniques discussed herein are also applicable to the other methods of performing thermometry with magnetic resonance imaging.

An 'ultrasound window' as used herein encompasses a window which is able to transmit ultrasonic waves or energy. Typically a thin film or membrane is used as an ultrasound window. The ultrasound window may for example be made of a thin membrane of BoPET (Biaxially-oriented polyethylene terephthalate).

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrated a method of operating the medical system of Fig. 1;
Fig. 3 illustrates a further example of a medical system;
Fig. 4 illustrates a further example of a medical system;
Fig. 5 illustrates a further example of a medical system; and
Fig. 6 shows a mapping of the electrical conductivity before and after a HIFU ablation.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

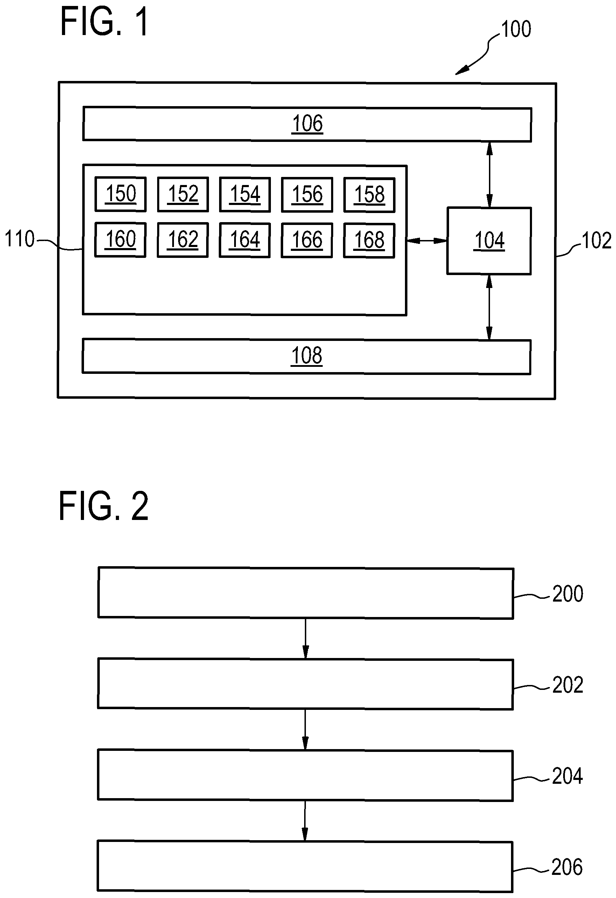

Fig. 1 illustrates an example of a medical system 100. In this example the medical system 100 comprises a computer 102 which has at least one processor 104. The processor is connected to a hardware interface 106, a user interface, and a memory 110. The optional user interface 106 may be used to connect the computer 102 to other pieces of equipment, hardware or computers for exchanging information or for controlling these other devices. The user interface 108 may enable a user to interact with and/or control the processor 104. The memory 110 may be any sort of memory or combination of memories which are accessible to a processor 104. This may include such things as main memory, cached memory, and also non-volatile memory such as flash RAM, hard drives, or other storage devices. In some examples the memory 110 may be considered to be a non-transitory computer-readable medium.

The memory 110 is shown as containing machine-executable instructions 150. The machine-executable instructions 150 enable the processor 140 to control other pieces of equipment and/or to perform operations on data. The memory 110 is further shown as containing first electric properties tomography data 152 and second electric properties tomography data 154. The first electric properties tomography data is descriptive of a first spatially dependent mapping of an electric property within a region of interest of a subject. The electric property is real valued permittivity and/or real valued conductivity. The second electric properties tomography data is also descriptive of a second spatially dependent mapping of the spatially dependent RF electrical property within the region of interest of the subject.

The memory 110 is shown as optionally containing a first spatially dependent temperature map 156 and a second spatially dependent temperature map 158. The first spatially dependent temperature map is descriptive of the temperature of the region of interest within a predetermined time period of when the first electric properties tomography data was acquired. The second spatially dependent temperature map 158 is descriptive of the temperature of the region of interest of the second electric properties tomography data 154 also within a predetermined time interval or period. The computer memory 110 is further shown as containing a mapping of a change in the spatially dependent RF electrical property 160 that was calculated using the first electric properties tomography data 152 and the second electric properties tomography data 154.

The memory 110 is further shown as containing a predetermined threshold 162. The memory 110 is further shown as containing a spatially dependent ablation map 164 which was created by thresholding the mapping of change 160 in the spatially dependent RF electrical property with the predetermined threshold 162.

The memory 110 is further shown as containing an optional first spatially dependent mapping 166 of an RF electrical property that was calculated from the first electric properties tomography data 152. The memory 110 is further shown as containing a second spatially dependent mapping 168 of the RF electrical property that was calculated from the second electric properties tomography data 154.

In some instances, the first spatially dependent temperature map 156 and the second spatially dependent temperature map 158 may be used for correcting the mapping 160. In some examples the mapping 160 is calculated directly from the first electric properties tomography data 152 and the second electric properties tomography data 154. In other instances the first electric properties tomography data 152 is first used to calculate the first spatially dependent mapping and the second electric properties tomography data 154 is used to calculate the second spatially dependent mapping. In this case the first spatially dependent mapping and the second spatially dependent mapping are used to calculate the mapping 160 of change in the spatially dependent RF electrical property.

Fig. 2 shows a flowchart which illustrates a method of operating the medical system 100 illustrated in Fig. 1. First in step 200, the processor 104 receives 200 the first electric properties tomography data 152. Next in step 202, the processor receives the second electric properties tomography data 154. Then in step 204, the processor calculates a change or mapping 160 of change in the spatially dependent RF electrical property. In step 206, the processor 104 calculates a spatially dependent ablation map 164 by indicating regions within the region of interest where the change in the spatially dependent RF electrical property is above the predetermined threshold 162.

In a modification to the method shown in Fig. 2 the processor may also receive the first spatially dependent temperature map 156 and the second spatially dependent temperature map 158 and then use this data to correct the mapping 160 of change in spatially dependent RF electrical property.

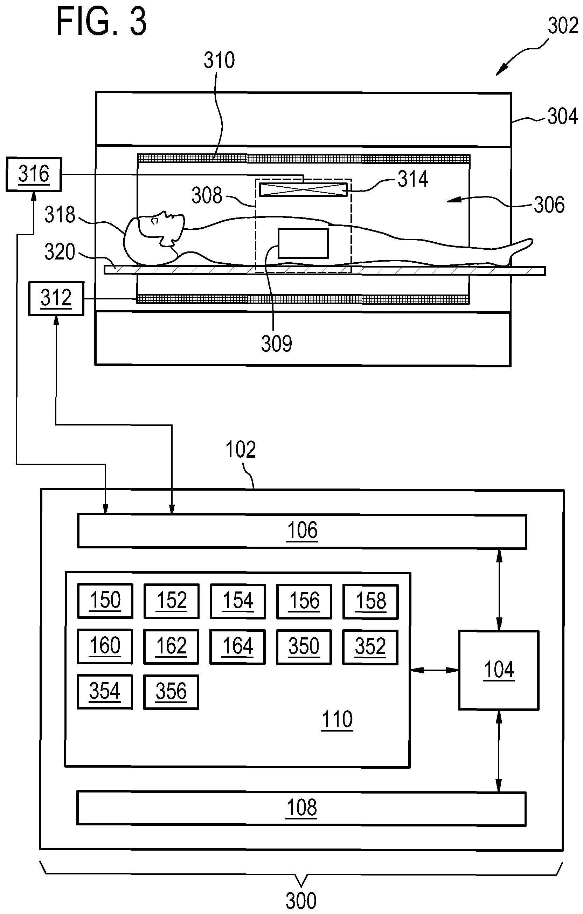

Fig. 3 shows a further example of a medical system 300. The medical system 300 is similar to that shown in Fig. 1 except that it additionally comprises a magnetic resonance imaging system 302. (note to self: insert standard text here)

The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 306 of the cylindrical magnet 304 there is an imaging zone 308 where the magnetic field is strong and uniform enough to perform magnetic resonance imaging. A region of interest 309 is shown within the imaging zone 308. A subject 318 is shown as being supported by a subject support 320 such that at least a portion of the subject 318 is within the imaging zone 308 and the region of interest 309.

Within the bore 306 of the magnet there is also a set of magnetic field gradient coils 310 which is used for acquisition of magnetic resonance data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels. For example if a parallel imaging technique such as SENSE is performed, the radio-frequency could 314 will have multiple coil elements.

The transceiver 316 and the gradient controller 312 are shown as being connected to a hardware interface 106 of a computer system 102.

The memory 110 is further shown as containing EPT pulse sequence commands 350. The EPT pulse sequence commands 350 are configured for controlling the magnetic resonance imaging system 302 to acquire the first electric properties tomography data 152 and the second electric properties tomography data 154. The EPT pulse sequence commands are configured to acquire the electric properties tomography data 152 and 154 according to an electrical properties tomography magnetic resonance imaging protocol.

The memory 110 is shown as optionally containing temperature sensitive pulse sequence commands 352 which enable the magnetic resonance imaging system to perform magnetic resonance thermometry. The temperature sensitive pulse sequence commands are configured for acquiring the first 354 thermal magnetic resonance data and second 356 thermal magnetic resonance data according to a magnetic resonance imaging thermometry protocol. The first thermal magnetic resonance data 354 is associated with the first electric properties tomography data 152. The second thermal magnetic resonance data 356 is associated with the second electric properties tomography data 154. The first thermal magnetic resonance data 354 is used to reconstruct the first 156 spatially dependent temperature map. The second thermal magnetic resonance data 356 is used to reconstruct the second 158 spatially dependent temperature map.

The medical system 300 shown in Fig. 3 may be implemented using a conventional magnetic resonance imaging system. For example the subject 318 could be imaged prior to a thermal ablation procedure and then imaged after the procedure has been finished. In some examples the processor 104 may be further configured to register the first electric properties tomography data 152 to the second electric properties tomography data 154 such that the position of the subject 318 can be corrected for. For example there may be preliminary or scouting images which are acquired to each of the data 152 and 154 which enables them to be accurately registered to each other.

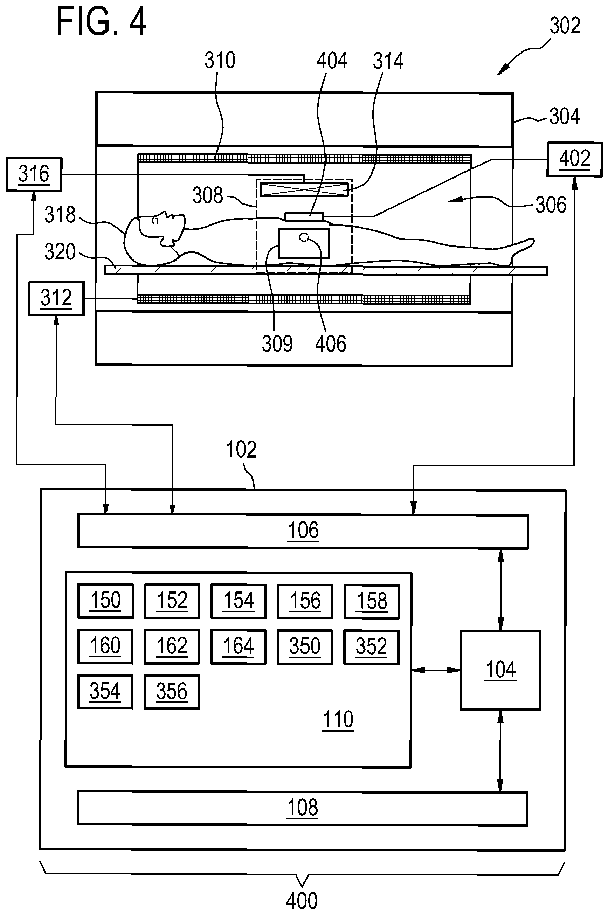

Fig. 4 shows a further example of a medical system 400. The medical system 400 is similar to that shown in Fig. 3 except there is additionally a tissue heating system 402. The tissue heating system 402 is shown to comprise an applicator 404. Items 402 and 404 are intended to be representative and may not necessarily depict all of the features of the particular embodiment. For example the tissue heating system 402 may become but is not limited to: a high-intensity focused ultrasound heating system, a radio-frequency heating system, a microwave ablation system, a hyperthermia therapy system, a laser ablation system, and an infrared ablation system. The applicator 404 may take different forms in different embodiments. It may be a heat exchanger, an infrared source, a laser source, a probe, a catheter, or even an antenna. The applicator 404 may in some instances be fixed with respect to its position in the magnet 304 or the subject support 320.

In other examples it may be mounted on or in the subject 318. In the example shown in Fig. 4 the subject 318 is lying in the magnetic resonance imaging system 302 and the thermal ablation can be performed using the tissue heating system 402. The first thermal magnetic resonance data 354 can be acquired before using the tissue heating system 402 and the second thermal magnetic resonance data 356 can be acquired after using the tissue heating system 402. In some instances the thermal magnetic resonance imaging may also be used to provide for the first spatially dependent temperature map 156 and the second spatially dependent temperature map 158 to correct the mapping 160.

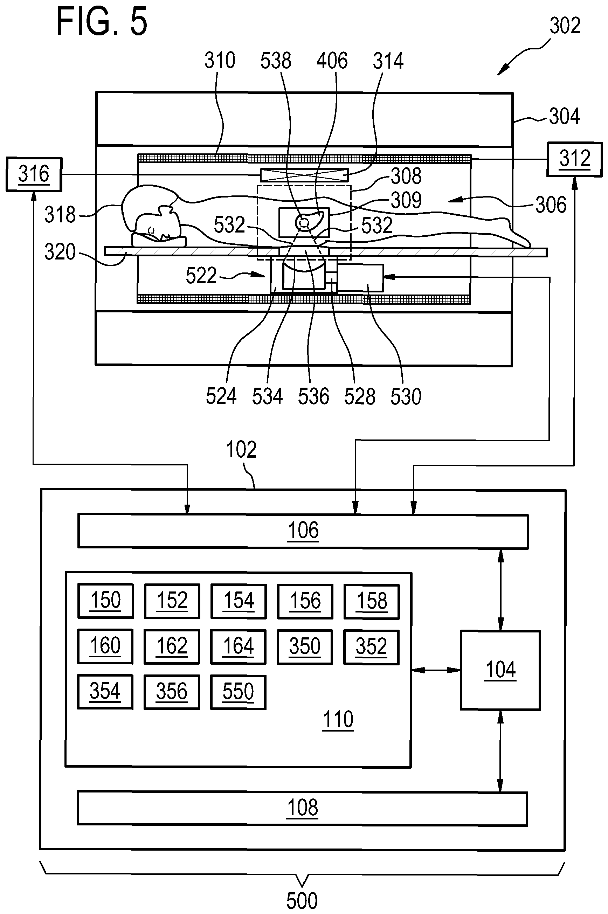

Fig. 5 illustrates a further example of a medical system 500. The medical system 500 is similar to the medical system 400 shown in Fig. 4. In Fig. 5 the tissue heating system is specifically a high-intensity focused ultrasound system 522.

A subject 318 is shown as reposing on a subject support 320 and is located partially within the imaging zone 308. The embodiment shown in Fig. 3 comprises a high-intensity focused ultrasound system 522. The high-intensity focused ultrasound system comprises a fluid-filled chamber 524. Within the fluid-filled chamber 524 is an ultrasound transducer 526. Although it is not shown in this figure the ultrasound transducer 526 may comprise multiple ultrasound transducer elements each capable of generating an individual beam of ultrasound. This may be used to steer the location of a sonication point 538 (the controllable focus) electronically by controlling the phase and/or amplitude of alternating electrical current supplied to each of the ultrasound transducer elements.

The ultrasound transducer 526 is connected to a mechanism 528 which allows the ultrasound transducer 526 to be repositioned mechanically. The mechanism 528 is connected to a mechanical actuator 530 which is adapted for actuating the mechanism 528. The mechanical actuator 530 also represents a power supply for supplying electrical power to the ultrasound transducer 526. In some embodiments the power supply may control the phase and/or amplitude of electrical power to individual ultrasound transducer elements. In some embodiments the mechanical actuator/power supply 530 is located outside of the bore 506 of the magnet 504.

The ultrasound transducer 526 generates ultrasound which is shown as following the path 532. The ultrasound 532 goes through the fluid-filled chamber 528 and through an ultrasound window 534. In this embodiment the ultrasound then passes through a gel pad 536. The gel pad 536 is not necessarily present in all embodiments but in this embodiment there is a recess in the subject support 520 for receiving a gel pad 536. The gel pad 536 helps couple ultrasonic power between the transducer 526 and the subject 518. After passing through the gel pad 536 the ultrasound 532 passes through the subject 518 and is focused to a sonication point 538 within a target volume 406. The sonication point 406 is being focused within a target volume 406. The sonication point 538 may be moved through a combination of mechanically positioning the ultrasonic transducer 426 and electronically steering the position of the sonication point 338 to treat the entire target volume 340.

The the high-intensity focused ultrasound system 522 are shown as being connected to the hardware interface 106 of computer 102.

The memory 110 is further shown as containing sonication commands 550. The sonication commands 550 are commands which enable the processor 104 to control the high-intensity focused ultrasound system 522 to move the sonication point 406 or controllable focus to sonicate the target zone or volume 406.

In many practical applications the target zone 406 will be sonicated by performing a number of sonications which are interrupted by cooling periods. During the cooling periods, magnetic resonance thermometry or acquisition of the first 152 and second 154 electric properties tomography data could be acquired. This may enable direct measurement of ablated tissue within the subject 318 during the sonication process. In some examples the machine-executable instructions 150 may be configured such that the mapping 160 of the change in the spatially dependent RF electrical property is updated repeatedly and used to generate an updated spatially dependent ablation map 164. The spatially dependent ablation map 164 could be used to identify which portions of the target zone have actually been ablated and adjust the sonication commands 550 during the cooling periods. This may enable more accurate ablation of the target zone 406 and/or to perform the ablation more rapidly. Such modifications could be performed automatically by the processor 104 or they could be displayed during the cooling period for adjustment by a human operator.

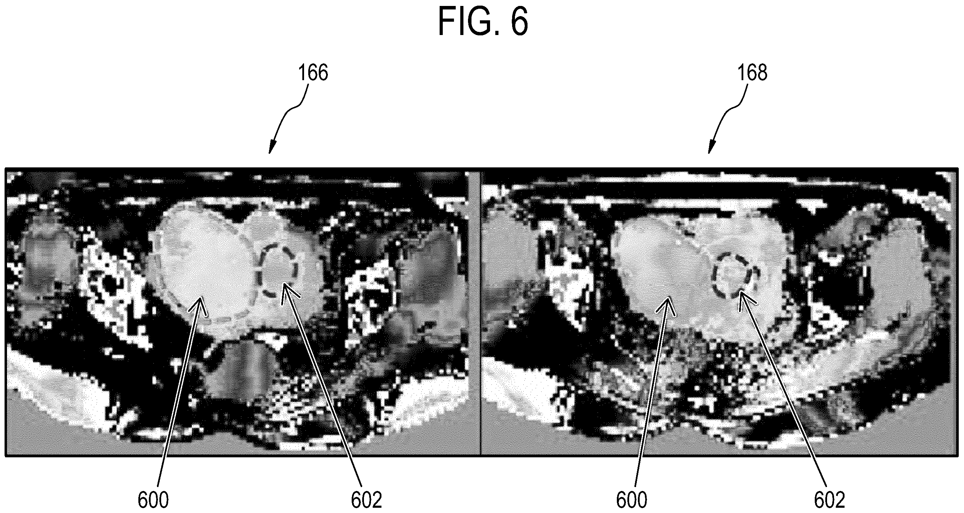

Fig. 6 below provides an example where ablated tissue was detected using EPT. In this example, 37-year-old female patient with multiple fibroids was treated with a volumetric 1.5 T MR-HIFU system. EPT was based on a balanced Fast Field Echo (bFFE) sequence (TR/TE=2.4/1.2 ms, voxel=2.5×2.5×2.5 mm³, flip=30°) acquired prior to and at 1.5 hours after MR-HIFU ablation. It is assumed that after this time, temperature of treated tissue is back to normal body temperature, and thus conductivity is not impacted by direct thermal effects. Conductivity reconstruction was performed using the phase-based approach of EPT and a subsequent bilateral median filter using tissue boundaries delineated from the bFFE magnitude image. The average conductivity was determined by drawing a region of interest around the whole index fibroid.

Fig. 6 shows an example of a first spatially dependent mapping 166 of an electric property in the form of a conductivity map. Fig. 6 also shows a second spatially dependent mapping 168 of an electric property. The electric property is again conductivity and the region of interest for images 166 and 168 are identical. Fig. 6 shows the average conductivities of the subserosal fibroid before and after sonication that were 1.02 S/m and 1.14 S/m respectively. Similarly the subserosal fibroid showed a 20.9% increase in conductivity from 1.10 S/m before to 1.33 S/m post treatment. The subserosal fibroid is the region labeled 600 and the submucosal fibroid is labeled 602. The images in Fig. 6 clearly demonstrate that a change in an RF electrical property such as the conductivity can be used to identify regions which have been ablated.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: medical system
- 102: computer
- 104: processor
- 106: hardware interface
- 108: user interface
- 110: memory
- 150: machine executable instructions
- 152: first electric properties tomography data
- 154: second electric properties tomography data
- 156: first spatially dependent temperature map
- 158: second spatially dependent temperature map
- 160: mapping of change in the spatially dependent RF electrical property
- 162: predetermined threshold
- 164: spatially dependent ablation map
- 166: first spatially dependent mapping of an RF electrical property
- 168: second spatially depenent mapping of an electircal property
- 200: receive first electric properties tomography data descriptive of a first spatially dependent mapping of an RF electrical property within a region of interest of a subject, wherein the RF electrical property is a real valued permittivity or real valued conductivity
- 202: receive second electric properties tomography data descriptive of a second spatially dependent mapping of the spatially dependent RF electrical property within the region of interest of the subject
- 204: calculate a change in the spatially dependent RF electrical property derived from a difference between the first spatially dependent mapping and the second spatially dependent mapping
- 206: calculate a spatially dependent ablation map by indicating regions within the region of interest where the change in the spatially dependent RF electrical property is above a predetermined threshold
- 300: medical system
- 302: magnetic resonance imaging system
- 304: magnet
- 306: bore of magnet
- 308: imaging zone
- 309: region of interest
- 310: magnetic field gradient coils
- 312: magnetic field gradient coil power supply
- 314: radio-frequency coil
- 316: transceiver
- 318: subject
- 320: subject support
- 350: EPT pulse sequence commands
- 352: temperature sensitive pulse sequence commands
- 354: first thermal magnetic resonance data
- 356: second thermal magnetic resonacne data
- 400: medical system
- 402: tissue heating system
- 404: applicator
- 406: target zone
- 500: medical system
- 522: high intensity focused ultrasound system
- 524: fluid filled chamber
- 526: ultrasound transducer
- 528: mechanism
- 530: mechanical actuator/power supply
- 532: path of ultrasound
- 534: ultrasound window
- 536: gel pad
- 538: controllable focus
- 550: sonication commands

## Claims

1. A medical system (100, 300, 400, 500) comprising:
- a memory (110) for storing machine executable instructions (150),
- a processor (104) for controlling the medical system, wherein execution of the machine executable instructions cause the processor to:
- receive (200) first electric properties tomography data (152) descriptive of a first spatially dependent mapping (166) of an RF electrical property within a region of interest (310) of a subject (318), wherein the RF electrical property is a real valued permittivity or real valued conductivity;
- receive (202) second electric properties tomography data (154) descriptive of a second spatially dependent mapping (168) of the spatially dependent RF electrical property within the region of interest of the subject;
- calculate (204) a change (160) in the spatially dependent RF electrical property derived from a difference between the first electric properties tomography data and the second electric properties tomography data; and
- calculate (206) a spatially dependent ablation map (164) by indicating regions within the region of interest where the change in the spatially dependent RF electrical property is above a predetermined threshold.

2. The medical system of claim 1, wherein the predetermined threshold is any one of the following: 5%, 10%, 20%, and between 5% and 20%.

3. The medical system of claim 1 or 2, wherein execution of the machine executable instructions further causes the processor to:
- receive a first spatially dependent temperature map (156) of the region of interest for the first electric properties tomography data; and
- receive a second spatially dependent temperature map (158) of the region of interest for the second electric properties tomography data; and
- wherein the change in the spatially dependent RF electrical property is temperature corrected using a change between the first spatially dependent temperature map and the second spatially dependent temperature map.

4. The medical system of claim 1 or 2, wherein the medical system further comprises a magnetic resonance imaging system (102), wherein the memory further stores EPT pulse sequence commands (350) configured for controlling the magnetic resonance imaging system to acquire the first electric properties tomography data and the second electric properties tomography data according to an electrical properties tomography magnetic resonance imaging protocol, wherein the first electric properties tomography data is received by controlling the magnetic resonance imaging system with the EPT pulse sequence commands, and wherein the second electric properties tomography data is received by controlling the magnetic resonance imaging system with the EPT pulse sequence commands.

5. The medical system of claim 4, wherein the magnetic resonance imaging system has an imaging zone (308), wherein the medical system further comprises a tissue heating system (402, 404) for heating a target zone (406) within the imaging zone, wherein the tissue heating system is configured for heating within the region of interest between the acquisition of the first electric properties tomography data and the second electric properties tomography data.

6. The medical system of claim 5, wherein the tissue heating system is any one of the following: a high intensity focused ultrasound heating system (522), radio-frequency heating system, a microwave ablation system, a hyperthermia therapy system, a laser ablation system, and an infrared ablation system.

7. The medical system of claim 5, wherein the tissue heating system is a high intensity focused ultrasound system (522) with a controllable focus (538) for depositing ultrasonic energy within the target zone, wherein the memory further comprises sonication commands (550) for controlling targeting of the controllable focus; wherein the sonication commands are configured for controlling the high intensity focused ultrasound system to sonicate the target zone in discrete sonication periods separated by cooling periods, wherein execution of the machine executable instructions further causes the processor to repeatedly acquire the first electric properties tomography data and the second electric properties tomography data during at least a portion of the cooling periods.

8. The medical system of claim 7, wherein execution of the machine executable instructions further causes the processor to modify the sonication commands using the spatially dependent ablation map after acquisition of the first electric properties tomography data and the second electric properties tomography data.

9. The medical system of any one of claims 4 through 7, wherein the memory further stores temperature sensitive pulse sequence commands (352) configured for controlling the magnetic resonance imaging system to acquire first thermal magnetic resonance data (354) and second thermal magnetic resonance data (356) according to a magnetic resonance imaging thermometry protocol, wherein execution of the machine executable instructions further causes the processor to:
- control the magnetic resonance imaging system to acquire the first thermal magnetic resonance data using the temperature sensitive pulse sequence commands, wherein the first thermal magnetic resonance data is acquired within a predetermined period of when the first electric properties tomography data is acquired, wherein the first spatially dependent temperature map is received by reconstructing the first spatially dependent temperature map from the first thermal magnetic resonance data; and
- control the magnetic resonance imaging system to acquire the second thermal magnetic resonance data using the temperature sensitive pulse sequence commands, wherein the second thermal magnetic resonance data is acquired within a predetermined period of when the second electric properties tomography data is acquired, wherein the second spatially dependent temperature map is received by reconstructing the second spatially dependent temperature map from the second thermal magnetic resonance data.

10. The medical system of any one of the preceding claims, wherein the spatially dependent RF electrical property is determined at a frequency between 1 MHz and 3 GHz or between 10 MHz and 500 MHz

11. A method of operating a medical system (100, 300, 400, 500), wherein the method comprises:
- receiving (200) first electric properties tomography data (152) descriptive of a first spatially dependent mapping (!66) of an RF electrical property within a region of interest (310) of a subject (318), wherein the RF electrical property is a real valued permittivity or real valued conductivity;
- receiving (202) second electric properties tomography data (154) descriptive of a second spatially dependent mapping (168) of the spatially dependent RF electrical property within the region of interest;
- calculating (204) a change (160) in the spatially RF dependent electrical property derived from a difference between the first electric properties tomography data and the second electric properties tomography data; and
- calculating (206) a spatially dependent ablation map (164) by indicating regions within the region of interest where the change in the spatially dependent RF electrical property is above a predetermined threshold.

12. A computer program product comprising machine executable instructions (150) for execution by a processor (104) controlling a medical instrument (100, 300, 400, 500), wherein execution of the machine executable instructions cause the processor to:
- receive (200) first electric properties tomography data (152) descriptive of a first spatially dependent mapping (166) of an RF electrical property within a region of interest (310) of a subject (318), wherein the RF electrical property is a real valued permittivity or real valued conductivity;
- receive (202) second electric properties tomography data (154) descriptive of a second spatially dependent mapping (168) of the spatially dependent RF electrical property within the region of interest of the subject;
- calculate (204) a change (160) in the spatially dependent RF electrical property derived from a difference between the first electric properties tomography data and the second electric properties tomography data; and
- calculate (206) a spatially dependent ablation map (164) by indicating regions within the region of interest where the change in the spatially dependent RF electrical property is above a predetermined threshold.

13. The computer program product of claim 12, wherein the medical system further comprises a magnetic resonance imaging system (302), wherein the first electric properties tomography data is received by controlling the magnetic resonance imaging system with EPT pulse sequence commands (350), wherein the EPT pulse sequence commands configured for controlling the magnetic resonance imaging system to acquire the first electric properties tomography data and the second electric properties tomography data according to an electrical properties tomography magnetic resonance imaging protocol, and wherein the second electric properties tomography is received by controlling the magnetic resonance imaging system with the EPT pulse sequence commands.

14. The computer program product of claim 13, wherein the medical instrument further comprises a high intensity focused ultrasound system (522) with a controllable focus (538) for depositing ultrasonic energy within the target zone, wherein the target zone is within the imaging zone, wherein the high intensity focused ultrasound is configured for heating within the region of interest between the acquisition of the first electric properties tomography data and the second electric properties tomography data, wherein the memory further comprises sonication commands for controlling targeting of the controllable focus, wherein the sonication commands are configured for controlling the high intensity focused ultrasound system to sonicate the target zone in discrete sonication periods separated by cooling periods, wherein execution of the machine executable instructions further causes the processor to repeatedly acquire the first electric properties tomography data and the second electric properties tomography data during at least a portion of the cooling periods.

## Patentansprüche

1. Medizinisches System (100, 300, 400, 500), das aus Folgendem besteht:
- einem Speicher (110) zum Speichern von maschinenausführbaren Befehlen (150),
- einen Prozessor (104) zur Steuerung des medizinischen Systems, wobei die Ausführung der maschinenausführbaren Befehle den Prozessor dazu veranlasst:
- erste Tomographiedaten (152) einer elektrischen Eigenschaft zu empfangen (200), die eine erste räumlich abhängige Kartierung (166) einer elektrischen HF-Eigenschaft innerhalb eines interessierenden Bereichs (310) eines Patienten (318) beschreiben, wobei die elektrische HF-Eigenschaft eine reellwertige Permittivität oder eine reellwertige Leitfähigkeit ist;
- zweite Tomographiedaten (154) einer elektrischen Eigenschaft zu erhalten (202), die eine zweite räumlich abhängige Kartierung (168) der räumlich abhängigen elektrischen HF-Eigenschaft innerhalb des interessierenden Bereichs des Patienten beschreiben;
- eine Änderung (160) in der räumlich abhängigen elektrischen HF-Eigenschaft zu berechnen (204), die aus einer Differenz zwischen den Tomographiedaten der ersten elektrischen Eigenschaften und den Tomographiedaten der zweiten elektrischen Eigenschaften abgeleitet wird; und
eine räumlich abhängige Ablationskarte (164) zu berechnen (206), indem Bereiche innerhalb des interessierenden Bereiches angegeben werden, in denen die Änderung der räumlich abhängigen elektrischen HF-Eigenschaft über einem vorbestimmten Schwellenwert liegt.

2. Medizinisches System von Anspruch 1, wobei der vorgegebene Schwellenwert einer der folgenden ist: 5 %, 10 %, 20 % und zwischen 5 % und 20 %.

3. Medizinisches System nach Anspruch 1 oder 2, bei dem die Ausführung der maschinenausführbaren Anweisungen den Prozessor ferner dazu veranlasst:
- eine erste räumlich abhängige Temperaturkarte (156) des Bereichs zu erhalten, der für die ersten Tomographiedaten über elektrische Eigenschaften von Interesse ist; und
- eine zweite räumlich abhängige Temperaturkarte (158) des Bereichs zu erhalten, der für die Tomographiedaten der zweiten elektrischen Eigenschaften von Interesse ist; und
- wobei die Änderung in der räumlich abhängigen elektrischen HF-Eigenschaft temperaturkorrigiert wird, indem eine Änderung zwischen der ersten räumlich abhängigen Temperaturkarte und der zweiten räumlich abhängigen Temperaturkarte verwendet wird.

4. Medizinisches System nach Anspruch 1 oder 2, wobei das medizinische System ferner ein Magnetresonanz-Bildgebungssystem (102) umfasst, wobei der Speicher ferner EPT-Impulsfolgebefehle (350) speichert, die zum Steuern des Magnetresonanz-Bildgebungssystems ausgelegt sind, um die Tomographiedaten der ersten elektrischen Eigenschaften und die Tomographiedaten der zweiten elektrischen Eigenschaften gemäß einem Magnetresonanz-Bildgebungsprotokoll für die Tomographie der elektrischen Eigenschaften zu erfassen, wobei die Tomographiedaten der ersten elektrischen Eigenschaften durch Steuern des Magnetresonanz-Bildgebungssystems mit den EPT-Impulsfolgebefehlen empfangen werden, und wobei die Tomographiedaten der zweiten elektrischen Eigenschaften durch Steuern des Magnetresonanz-Bildgebungssystems mit den EPT-Impulsfolgebefehlen empfangen werden.

5. Medizinisches System nach Anspruch 4, wobei das Magnetresonanz-Bildgebungssystem einen Abbildungsbereich (308) aufweist, wobei das medizinische System ferner ein Gewebe-Erwärmungssystem (402, 404) zum Erwärmen eines Zielbereichs (406) innerhalb des Abbildungsbereichs umfasst, wobei das Gewebeheizsystem zum Erwärmen innerhalb des interessierenden Bereichs zwischen der Erfassung der Tomographiedaten der ersten elektrischen Eigenschaften und der Tomographiedaten der zweiten elektrischen Eigenschaften ausgelegt ist.

6. Medizinisches System nach Anspruch 5, wobei das Gewebeerwärmungssystem eines der folgenden ist: ein hochintensives fokussiertes Ultraschall-Erwärmungssystem (522), ein Hochfrequenz-Erwärmungssystem, ein Mikrowellen-Ablationssystem, ein Hyperthermie-Therapiesystem, ein Laser-Ablationssystem oder ein Infrarot-Ablationssystem.

7. Medizinisches System nach Anspruch 5, wobei das Gewebe-Erwärmungssystem ein hochintensives fokussiertes Ultraschallsystem (522) mit einem steuerbaren Fokus (538) zur Ablagerung von Ultraschallenergie innerhalb des Zielberichs ist, wobei der Speicher ferner Beschallungsbefehle (550) zur Steuerung des Zielens des steuerbaren Fokus umfasst; wobei die Beschallungsbefehle zum Steuern des fokussierten Ultraschallsystems mit hoher Intensität ausgelegt sind, um den Zielbereich in diskreten Beschallungsperioden zu beschallen, die durch Kühlperioden getrennt sind, wobei die Ausführung der maschinenausführbaren Befehle ferner bewirkt, dass der Prozessor wiederholt die Tomographiedaten der ersten elektrischen Eigenschaften und die Tomographiedaten der zweiten elektrischen Eigenschaften während mindestens eines Teils der Kühlperioden erfasst.

8. Medizinisches System nach Anspruch 7, bei dem die Ausführung der maschinell ausführbaren Befehle den Prozessor ferner dazu veranlasst, die Beschallungsbefehle unter Verwendung der räumlich abhängigen Ablationskarte nach Erfassung der ersten Tomographiedaten einer elektrischen Eigenschaft und der zweiten Tomographiedaten einer elektrischen Eigenschaft zu modifizieren.

9. Medizinisches System nach einem der Ansprüche 4 bis 7, wobei der Speicher ferner temperaturempfindliche Impulsfolgebefehle (352) speichert, die zur Steuerung des Magnetresonanz-Bildgebungssystems ausgelegt sind, um erste thermische Magnetresonanzdaten (354) und zweite thermische Magnetresonanzdaten (356) gemäß einem Magnetresonanz-Bildgebungs-Thermometrieprotokoll zu erfassen, wobei die Ausführung der maschinenausführbaren Befehle ferner den Prozessor dazu veranlasst:
- das Magnetresonanz-Bildgebungssystems zu steuern, um die ersten thermischen Magnetresonanzdaten unter Verwendung der temperaturempfindlichen Impulsfolgebefehle zu erfassen, wobei die ersten thermischen Magnetresonanzdaten innerhalb einer vorbestimmten Periode erfasst werden, wenn die ersten Tomographiedaten einer elektrischen Eigenschaft erfasst werden, wobei die erste räumlich abhängige Temperaturkarte durch Rekonstruktion der ersten räumlich abhängigen Temperaturkarte aus den ersten thermischen Magnetresonanzdaten erhalten wird; und
- das Magnetresonanz-Bildgebungssystems zu steuern, um die zweiten thermischen Magnetresonanzdaten unter Verwendung der temperaturempfindlichen Impulsfolgebefehle zu erfassen, wobei die zweiten thermischen Magnetresonanzdaten innerhalb einer vorbestimmten Periode erfasst werden, wenn die zweiten Tomographiedaten einer elektrischen Eigenschaft erfasst werden, wobei die zweite räumlich abhängige Temperaturkarte durch Rekonstruktion der zweiten räumlich abhängigen Temperaturkarte aus den zweiten thermischen Magnetresonanzdaten erhalten wird.

10. Medizinisches System nach einem der vorhergehenden Ansprüche, wobei die räumlich abhängige elektrische HF-Eigenschaft bei einer Frequenz zwischen 1 MHz und 3 GHz oder zwischen 10 MHz und 500 MHz bestimmt wird

11. Verfahren zum Betreiben eines medizinischen Systems (100, 300, 400, 500), wobei das Verfahren Folgendes umfasst:
- Empfang (200) erster Tomographiedaten (152) elektrischer Eigenschaften, die eine erste räumlich abhängige Abbildung (166) einer elektrischen HF-Eigenschaft innerhalb eines interessierenden Bereichs (310) eines Subjekts (318) beschreiben, wobei die elektrische HF-Eigenschaft eine reellwertige Permittivität oder eine reellwertige Leitfähigkeit ist;
- Empfang (202) zweiter Tomographiedaten (154) elektrischer Eigenschaften, die eine zweite räumlich abhängige Kartierung (168) der räumlich abhängigen elektrischen HF-Eigenschaft innerhalb des Bereichs von Interesse beschreiben;
- Berechnung (204) einer Änderung (160) in der räumlich HF-abhängigen elektrischen Eigenschaft, die aus einer Differenz zwischen den Tomographiedaten der ersten elektrischen Eigenschaften und den Tomographiedaten der zweiten elektrischen Eigenschaften abgeleitet wird; und
- Berechnung (206) einer räumlich abhängigen Ablationskarte (164) durch Angabe von Regionen innerhalb des Bereichs von Interesse, in denen die Änderung der räumlich abhängigen elektrischen HF-Eigenschaft über einem vorbestimmten Schwellenwert liegt.

12. Computerprogramm mit maschinenausführbaren Befehlen (150) zur Ausführung durch einen Prozessor (104), der ein medizinisches Instrument (100, 300, 400, 500) steuert, wobei die Ausführung der maschinenausführbaren Befehle den Prozessor dazu veranlasst:
- erste Tomographiedaten (152) einer elektrischen Eigenschaft zu empfangen (200), die eine erste räumlich abhängige Kartierung (166) einer elektrischen HF-Eigenschaft innerhalb eines interessierenden Bereichs (310) eines Patienten (318) beschreiben, wobei die elektrische HF-Eigenschaft eine reellwertige Permittivität oder eine reellwertige Leitfähigkeit ist;
- zweite Tomographiedaten (154) einer elektrischen Eigenschaft zu erhalten (202), die eine zweite räumlich abhängige Kartierung (168) der räumlich abhängigen elektrischen HF-Eigenschaft innerhalb des interessierenden Bereichs des Patienten beschreiben;
- eine Änderung (160) in der räumlich abhängigen elektrischen HF-Eigenschaft zu berechnen (204), die aus einer Differenz zwischen den Tomographiedaten der ersten elektrischen Eigenschaften und den Tomographiedaten der zweiten elektrischen Eigenschaften abgeleitet wird; und
- eine räumlich abhängige Ablationskarte (164) zu berechnen (206), indem Bereiche innerhalb des interessierenden Bereiches angegeben werden, in denen die Änderung der räumlich abhängigen elektrischen HF-Eigenschaft über einem vorbestimmten Schwellenwert liegt.

13. Computerprogramm nach Anspruch 12, wobei das medizinische System ferner ein Magnetresonanz-Bildgebungssystem (302) umfasst, wobei die Tomographiedaten der ersten elektrischen Eigenschaften durch Steuerung des Magnetresonanz-Bildgebungssystems mit EPT-Impulsfolgebefehlen (350) empfangen werden, wobei die EPT-Impulsfolgebefehle zur Steuerung des Magnetresonanz-Bildgebungssystems dazu ausgelegt sind, die Tomographiedaten der ersten elektrischen Eigenschaften und die Tomographiedaten der zweiten elektrischen Eigenschaften nach einem Magnetresonanztomographie-Protokoll für elektrische Eigenschaften, zu erdfassen, und
wobei die Tomographie der zweiten elektrischen Eigenschaften durch Steuerung des Magnetresonanz-Bildgebungssystems mit den EPT-Impulsfolgebefehlen erhalten wird.

14. Computerprogramm nach Anspruch 13, wobei das medizinische Instrument ferner ein fokussiertes Ultraschallsystem (522) mit hoher Intensität mit einem steuerbaren Fokus (538) zum Abgeben von Ultraschallenergie innerhalb des Zielbereichs umfasst, wobei der Zielbereich innerhalb des Bildgebungsbereichs liegt, wobei der fokussierte Ultraschall hoher Intensität zum Erwärmen innerhalb des interessierenden Bereichs zwischen der Erfassung der Tomographiedaten der ersten elektrischen Eigenschaften und der Tomographiedaten der zweiten elektrischen Eigenschaften ausgelegt ist, wobei der Speicher ferner Beschallungsbefehle zur Steuerung des Zielens des steuerbaren Fokus umfasst, wobei die Beschallungsbefehle zur Steuerung des fokussierten Hochintensitäts-Ultraschallsystems konfiguriert sind, um den Zielbereich in diskreten Beschallungsperioden zu beschallen, die durch Kühlperioden getrennt sind, wobei die Ausführung der maschinenausführbaren Befehle ferner bewirkt, dass der Prozessor wiederholt die Tomographiedaten der ersten elektrischen Eigenschaften und die Tomographiedaten der zweiten elektrischen Eigenschaften während mindestens eines Teils der Kühlperioden erfasst.

## Revendications

1. Système médical (100, 300, 400, 500) comprenant :
- une mémoire (110) pour mémoriser des instructions exécutables par machine (150),
- un processeur (104) pour commander ledit système médical, dans lequel l'exécution des instructions exécutables par machine amène ledit processeur :
- à recevoir (200) des premières données de tomographie de propriétés électriques (152) descriptives d'une première cartographie (166) spatialement dépendante d'une propriété électrique RF dans une zone d'intérêt (310) d'un sujet (318), dans lequel ladite propriété électrique RF est une permittivité à valeur réelle ou une conductivité à valeur réelle ;
- à recevoir (202) des secondes données de tomographie de propriétés électriques (154) descriptives d'une seconde cartographie (168) spatialement dépendante de la propriété électrique RF spatialement dépendante dans la zone d'intérêt du sujet ;
- à calculer (204) un changement (160) dans la propriété électrique RF spatialement dépendante dérivée d'une différence entre les premières données de tomographie de propriétés électriques et les secondes données de tomographie de propriétés électriques ; et
- à calculer (206) une carte d'ablation (164) spatialement dépendante par indication des zones dans la zone d'intérêt, dans lesquelles le changement de la propriété électrique RF spatialement dépendante est supérieur à un seuil prédéterminé.

2. Système médical selon la revendication 1, dans lequel le seuil prédéterminé est l'un quelconque des seuils suivants : 5 %, 10 %, 20 % et entre 5 % et 20 %.

3. Système médical selon la revendication 1 ou 2, dans lequel l'exécution des instructions exécutables par machine amène en outre le processeur :
- à recevoir une première carte de température (156) spatialement dépendante de la zone d'intérêt pour les premières données de tomographie de propriétés électriques ; et
- à recevoir une seconde carte de température (158) spatialement dépendante de la zone d'intérêt pour les secondes données de tomographie de propriétés électriques ; et
- dans lequel le changement de la propriété électrique RF spatialement dépendante est corrigé en température à l'aide d'un changement entre la première carte de température spatialement dépendante et la seconde carte de température spatialement dépendante.

4. Système médical selon la revendication 1 ou 2, dans lequel ledit système médical comprend en outre un système d'imagerie par résonance magnétique (102), dans lequel la mémoire mémorise en outre des commandes de séquence d'impulsions EPT (350) configurées pour commander le système d'imagerie par résonance magnétique pour acquérir les premières données de tomographie de propriétés électriques et les secondes données de tomographie de propriétés électriques selon un protocole d'imagerie par résonance magnétique de la tomographie de propriétés électriques, dans lequel les premières données de tomographie de propriétés électriques sont reçues par commande du système d'imagerie par résonance magnétique au moyen des commandes de séquence d'impulsions EPT, et dans lequel les secondes données de tomographie de propriétés électriques sont reçues par commande du système d'imagerie par résonance magnétique à l'aide des commandes de séquence d'impulsions EPT.

5. Système médical selon la revendication 4, dans lequel le système d'imagerie par résonance magnétique comporte une zone d'imagerie (308), dans lequel le système médical comprend en outre un système de chauffage du tissu (402, 404) pour chauffer une zone cible (406) dans la zone d'imagerie, dans lequel le système de chauffage du tissu est conçu pour chauffer dans la zone d'intérêt entre l'acquisition des premières données de tomographie de propriétés électriques et les secondes données de tomographie de propriétés électriques.

6. Système médical selon la revendication 5, dans lequel le système de chauffage du tissu est l'un quelconque des systèmes de chauffage suivants : un système de chauffage par ultrasons focalisé à haute intensité (522), un système de chauffage par radiofréquence, un système d'ablation par micro-ondes, un système de thérapie par hyperthermie, un système d'ablation laser et un système d'ablation infrarouge.

7. Système médical selon la revendication 5, dans lequel le système de chauffage du tissu est un système à ultrasons focalisé à haute intensité (522) avec une mise au point (538) pouvant être commandée pour déposer de l'énergie ultrasonore dans la zone cible, dans lequel la mémoire comprend en outre des commandes de sonication (550) pour commander le ciblage de la mise au point pouvant être commandée ; dans lequel les commandes de sonication sont configurées pour commander le système à ultrasons focalisé à haute intensité pour soniquer la zone cible dans des périodes de sonication discrètes séparées par des périodes de refroidissement, dans lequel l'exécution des instructions exécutables par machine amène en outre le processeur à acquérir de manière répétée les premières données de tomographie de propriétés électriques et les secondes données de tomographie de propriétés électriques pendant au moins une partie des périodes de refroidissement.

8. Système médical selon la revendication 7, dans lequel l'exécution des instructions exécutables par machine amène en outre le processeur à modifier les commandes de sonication à l'aide de la carte d'ablation spatialement dépendante après l'acquisition des premières données de tomographie de propriétés électriques et des secondes données de tomographie de propriétés électriques.

9. Système médical selon l'une quelconque des revendications 4 à 7, dans lequel la mémoire mémorise en outre des commandes de séquence d'impulsions sensibles à la température (352) configurées pour commander le système d'imagerie par résonance magnétique pour acquérir des premières données de résonance magnétique thermique (354) et des secondes données de résonance magnétique thermique (356) selon un protocole de thermométrie de l'imagerie par résonance magnétique, dans lequel l'exécution des instructions exécutables par machine amène en outre le processeur :
- à commander le système d'imagerie par résonance magnétique pour acquérir les premières données de résonance magnétique thermique à l'aide des commandes de séquence d'impulsions sensibles à la température, dans lequel les premières données de résonance magnétique thermique sont acquises dans une période prédéterminée de l'acquisition des premières données de tomographie de propriétés électriques, dans lequel la première carte de température spatialement dépendante est reçue par reconstruction de la première carte de température spatialement dépendante à partir des premières données de résonance magnétique thermique ; et
- à commander le système d'imagerie par résonance magnétique pour acquérir les secondes données de résonance magnétique thermique à l'aide des commandes de séquence d'impulsions sensibles à la température, dans lequel les secondes données de résonance magnétique thermique sont acquises dans une période prédéterminée de l'acquisition des secondes données de tomographie de propriétés électriques, dans lequel la seconde carte de température spatialement dépendante est reçue par reconstruction de la seconde carte de température spatialement dépendante à partir des secondes données de résonance magnétique thermique.

10. Système médical selon l'une quelconque des revendications précédentes, dans lequel la propriété électrique RF spatialement dépendante est déterminée à une fréquence dans la plage comprise entre 1 MHz et 3 GHz ou entre 10 MHz et 500 MHz.

11. Procédé de fonctionnement d'un système médical (100, 300, 400, 500), dans lequel ledit procédé comprend :
- la réception (200) des premières données de tomographie de propriétés électriques (152) descriptives d'une première cartographie (166) spatialement dépendante d'une propriété électrique RF dans une zone d'intérêt (310) d'un sujet (318), dans lequel la propriété électrique RF est une permittivité à valeur réelle ou une conductivité à valeur réelle ;
- la réception (202) des secondes données de tomographie de propriétés électriques (154) descriptives d'une seconde cartographie (168) spatialement dépendante de la propriété électrique RF spatialement dépendante dans la zone d'intérêt ;
- le calcul (204) d'un changement (160) de la propriété électrique dépendante de l'espace RF dérivée d'une différence entre les premières données de tomographie de propriétés électriques et les secondes données de tomographie de propriétés électriques ; et
- le calcul (206) d'une carte d'ablation (164) spatialement dépendante par indication des zones dans la zone d'intérêt, dans lesquelles le changement de la propriété électrique RF spatialement dépendante est supérieur à un seuil prédéterminé.

12. Produit de programme informatique comprenant des instructions exécutables par machine (150) destinées à l'exécution par un processeur (104) commandant un instrument médical (100, 300, 400, 500), dans lequel l'exécution des instructions exécutables par machine amène le processeur :
- à recevoir (200) des premières données de tomographie de propriétés électriques (152) descriptives d'une première cartographie (166) spatialement dépendante d'une propriété électrique RF dans une zone d'intérêt (310) d'un sujet (318), dans lequel la propriété électrique RF est une permittivité à valeur réelle ou une conductivité à valeur réelle ;
- à recevoir (202) des secondes données de tomographie de propriétés électriques (154) descriptives d'une seconde cartographie (168) spatialement dépendante de la propriété électrique RF spatialement dépendante dans la zone d'intérêt du sujet ;
- à calculer (204) un changement (160) dans la propriété électrique RF spatialement dépendante dérivée d'une différence entre les premières données de tomographie de propriétés électriques et les secondes données de tomographie de propriétés électriques ; et
- à calculer (206) une carte d'ablation (164) spatialement dépendante par indication des zones dans la zone d'intérêt, dans lesquelles le changement de la propriété électrique RF spatialement dépendante est supérieur à un seuil prédéterminé.

13. Produit de programme informatique selon la revendication 12, dans lequel le système médical comprend en outre un système d'imagerie par résonance magnétique (302), dans lequel les premières données de tomographie de propriétés électriques sont reçues par commande du système d'imagerie par résonance magnétique à l'aide des commandes de séquence d'impulsions EPT (350), dans lequel les commandes de séquence d'impulsions EPT sont configurées pour commander le système d'imagerie par résonance magnétique pour acquérir les premières données de tomographie de propriétés électriques et les secondes données de tomographie de propriétés électriques selon un protocole d'imagerie par résonance magnétique de la tomographie de propriétés électriques, et dans lequel la seconde tomographie de propriétés électriques est reçue par commande du système d'imagerie par résonance magnétique à l'aide des commandes de séquence d'impulsions EPT.

14. Produit programme d'ordinateur selon la revendication 13, dans lequel l'instrument médical comprend en outre un système à ultrasons focalisé à haute intensité (522) avec une mise au point (538) pouvant être commandée pour déposer l'énergie ultrasonore dans la zone cible, dans lequel la zone cible se trouve dans de la zone d'imagerie, dans lequel les ultrasons focalisés à haute intensité sont conçus pour chauffer dans la zone d'intérêt entre l'acquisition des premières données de tomographie de propriétés électriques et les secondes données de tomographie de propriétés électriques, dans lequel la mémoire comprend en outre des commandes de sonication pour commander le ciblage de la mise au point pouvant être commandée, dans lequel les commandes de sonication sont configurées pour commander le système à ultrasons focalisé à haute intensité pour soniquer la zone cible dans des périodes de sonication discrètes séparées par des périodes de refroidissement, dans lequel l'exécution des instructions exécutables par machine amène en outre le processeur à acquérir de manière répétée les premières données de tomographie de propriétés électriques et les secondes données de tomographie de propriétés électriques pendant au moins une partie des périodes de refroidissement.
